(19) Europäisches Patentamt
European Patent Office
Office européen des brevets

(11) EP 1 172 442 A1

(12) **EUROPEAN PATENT APPLICATION**
published in accordance with Art. 158(3) EPC

(43) Date of publication:
**16.01.2002 Bulletin 2002/03**

(21) Application number: **00917304.8**

(22) Date of filing: **13.04.2000**

(51) Int Cl.[7]: **C12P 7/02**, C07C 401/00
// C12P7:02, C12R1:365,
C12P7:02, C12R1:465,
C12P7:02, C12R1:01

(86) International application number:
**PCT/JP00/02410**

(87) International publication number:
**WO 00/61776 (19.10.2000 Gazette 2000/42)**

(84) Designated Contracting States:
**AT BE CH CY DE DK ES FI FR GB GR IE IT LI LU MC NL PT SE**
Designated Extension States:
**AL LT LV MK RO SI**

(30) Priority: **14.04.1999 JP 10662299**

(71) Applicants:
- **MERCIAN CORPORATION**
  **Chuo-ku, Tokyo 104-8305 (JP)**
- **CHUGAI SEIYAKU KABUSHIKI KAISHA**
  **Tokyo, 115-8543 (JP)**

(72) Inventors:
- **TAKEDA, Koji**
  **Iwata-shi, Shizuoka 438-0078 (JP)**
- **KOMINATO, Kaichiro, Mercian Apartment, Room 122**
  **Iwata-shi, Shizuoka 438-0078 (JP)**
- **KUMAZAWA, Masateru**
  **Iwata-shi, Shizuoka 438-0078 (JP)**
- **SHIMIZU, Masato**
  **Tokyo 104-0014 (JP)**
- **YAMADA, Sachiko**
  **Hachioji-shi, Tokyo 193-0845 (JP)**

(74) Representative: **Horner, Martin Grenville et al**
**Cruikshank & Fairweather 19 Royal Exchange Square**
**Glasgow G1 3AE, Scotland (GB)**

(54) **VITAMIN D DERIVATIVES AND PROCESS FOR PRODUCING THE SAME**

(57) A process for producing hydroxyvitamin D derivatives, characterized by converting a hydrogen atom or atoms at the 2-position, 24-position, 25-position and/or 26-position of a vitamin D into a hydroxyl group or groups in a solution containing a microorganism that belongs to the genus *Nocardia*, *Streptomyces*, *Sphingomonas* or *Amycolata* which has an ability to hydroxylate vitamin Ds or an enzyme produced by that microorganism, and optionally under the coexistence of a cyclodextrin; and novel vitamin $D_3$ derivatives obtained by that process.

EP 1 172 442 A1

## Description

### TECHNICAL FIELD

The present invention relates to a biological hydroxylation process in which a hydrogen atom or atoms at the 2-position, 24-position, 25-position and/or 26-position of a vitamin D derivative are converted into a hydroxyl group or groups, and to novel vitamin $D_3$ derivatives produced by that process. More particularly, the invention relates to

(1) a process for producing vitamin D derivatives, characterized by converting a hydrogen atom or atoms at the 2-position, 24-position, 25-position and/or 26-position of a vitamin D derivative into a hydroxyl group or groups using a microorganism (a hydroxylation process) and to
(2) novel vitamin $D_3$ derivatives represented by the formula (I)

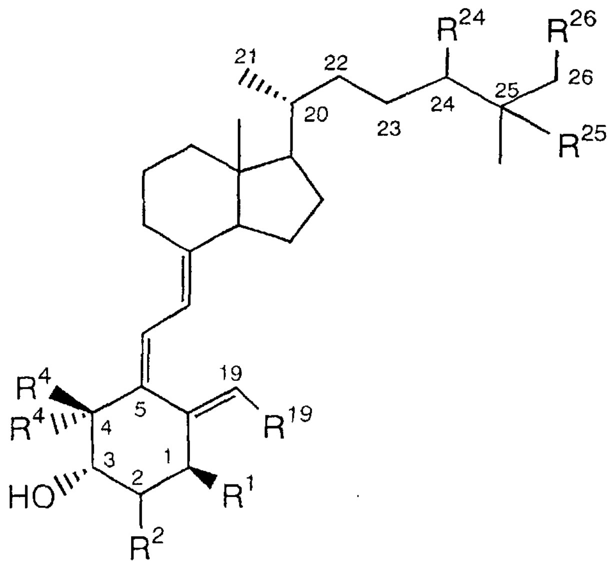

(I)

wherein all symbols have the same meanings as described later.

### BACKGROUND ART

Vitamin D derivatives are an important vitamin which relates to absorption of calcium, facilitation of bone calcium metabolism, or differentiation of epidermal keratinocytes *in vivo,* and it is known that their activity is exhibited upon hydroxylation at the 25-position in liver and at the 1α-position in kidney [H.F. Deluca and H.K. Jones: *Ann. Rev. Biochem*., Vol. 52, p.411 (1983)]. However, it is known that *in vivo* production quantity of 25-hydroxyvitamin $D_3$ is insufficient in patients with hepatic dysfunction, and administration of 25-hydroxyvitamin $D_3$ to these patients is effective in the light of making up *in vivo* shortfall. 1α,25-Dihydroxyvitamin $D_3$ reveals remarkable effects on patients with renal failure (rachitis with renal failure and the like) and osteoporosis.

While it is known that 1α,25-dihydroxyvitamin $D_3$ can be obtained from a raw material, 25-hydroxyvitamin $D_3$ by enzymatic chemical conversion using a microorganism, its conversion efficiency was not sufficient (Japanese Patent Laid-Open No. 469/1990 and No. 231089/1990 (U.S. Patent No. 4,892,821)).

Thus, the present inventors previously proposed a process for increasing the conversion efficiency under the coexistence of a cyclodextrin in the reaction solution in conversion process from vitamin D derivatives to 25-hydroxyvitamin D derivatives or 1α,25-dihydroxyvitamin D derivatives using a microorganism (Japanese Patent Laid-Open No. 166090/1992 (U.S. Patent No. 5,474,923)).

Moreover, in recent years, it is reported that a synthetic vitamin D derivative hydroxylated at the 1α- and 24-positions (1α,24-dihydroxyvitamin $D_3$) binds to a receptor specific for 1α,25-dihydroxyvitamin $D_3$ and shows similar activities to those of 1α,25-dihydroxyvitamin $D_3$ [*The Journal of Dermatology*, Vol. 17, p.135 (1990)].

Thus, various vitamin D derivatives are expected to enhance their original activities such as absorption of calcium, facilitation of bone calcium metabolism, differentiation of epidermal keratinocytes and the like, to augment selectivity, or to improve *in vivo* absorptivity.

DISCLOSURE OF THE INVENTION

The present inventors further studied on reaction solutions used for converting vitamin D derivatives into hydroxyvitamin D derivatives using microorganisms, which the present inventors previously developed. As a result, several kinds of novel hydroxyvitamin D derivative having unknown structures were confirmed. With respect to these novel compounds, while the details of their activities are being examined, they are expected to be new medicines having vitamin D actions.

The present inventors still further studied on the reaction solutions used in the process for producing vitamins hydroxylated at the 25-position of 1α-vitamin $D_3$ by the biological method as previously proposed (Japanese Patent Laid-Open No. 166090/1992 (U.S. Patent No. 5,474,923), examining vitamin $D_3$, 19-fluorovitamin $D_3$, 19-fluoro-1α-dihydroxyvitamin $D_3$, 4,4-difluorovitamin $D_3$, 4,4-difluoro-1α-hydroxyvitamin $D_3$, and 1α-hydroxyvitamin $D_3$ as substrates. As a result, finding novel hydroxyvitamin $D_3$ with hydroxyl groups being introduced therein at not only the 25-position but also the 2-position, 24-position and 26-position, they completed the present invention.

Specifically, the present invention provides:

(1) A biological process for producing hydroxyvitamin D derivatives, characterized by converting a hydrogen atom or atoms at the 2-position, 24-position, 25-position and/or 26-position of a vitamin D into a hydroxyl group or groups in a solution containing a microorganism which can hydroxylate vitamin Ds or an enzyme produced by that microorganism, and optionally under the coexistence of a cyclodextrin; and

(2) Vitamin $D_3$ derivatives represented by the formula (I):

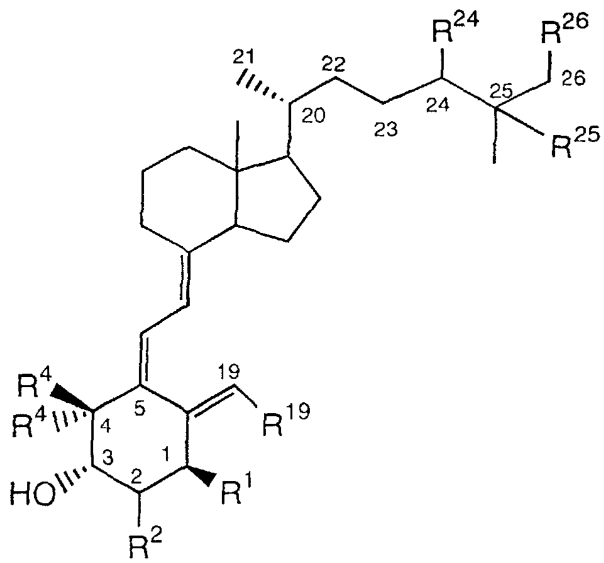

(I)

wherein $R^1$, $R^2$, $R^{24}$, $R^{25}$ and $R^{26}$ each independently represents a hydrogen atom or a hydroxyl group; and $R^4$ and $R^{19}$ each independently represents a hydrogen atom or a fluorine atom, provided that one of $R^2$, $R^{24}$ and $R^{26}$ is a hydroxyl group, and the others are each a hydrogen atom, provided that 1α,24-dihydroxyvitamin $D_3$ wherein $R^1$ and $R^{24}$ each represents a hydroxyl group, and $R^2$, $R^4$, $R^{19}$, $R^{25}$ and $R^{26}$ each represents a hydrogen atom is excluded.

DETAILED DESCRIPTION OF THE INVENTION

Specific examples of novel vitamin $D_3$ derivatives represented by the formula (I) include the following (1) to (10) compounds.

(1) 1α,26-Dihydroxyvitamin $D_3$
(2) 2α,25-Dihydroxyvitamin $D_3$
(3) 19-Fluoro-24-hydroxyvitamin $D_3$
(4) 19-Fluoro-26-hydroxyvitamin $D_3$
(5) 19-Fluoro-1α,24-dihydroxyvitamin $D_3$
(6) 19-Fluoro-1α,26-dihydroxyvitamin $D_3$
(7) 4,4-Difluoro-24-hydroxyvitamin $D_3$

(8) 4,4-Difluoro-26-hydroxyvitamin $D_3$
(9) 4,4-Difluoro-1α,24-dihydroxyvitamin $D_3$
(10) 4,4-Difluoro-1α,26-dihydroxyvitamin $D_3$

Vitamin Ds which are to be hydroxylated in the microbiological production process of the present invention are vitamin D derivatives having a hydrogen atom or atoms at the 2-position, 24-position, 25-position and/or 26-position.
Such vitamin D derivatives are, for example, vitamin $D_2$ derivatives, vitamin $D_3$ derivatives, vitamin $D_4$ derivatives, vitamin $D_5$ derivatives and vitamin $D_7$ derivatives, wherein hydrogen atom(s) or hydroxyl group(s) may be substituted with halogen atom(s) such as a fluorine atom, a hydroxyl group, a lower alkyl group or the like. Further, the two hydrogen atoms at the 4,4-positions and/or the hydrogen atom at the 19-position may be substituted with a halogen atom such as a fluorine atom.
Specific examples include:

vitamin $D_2$,
vitamin $D_3$,
vitamin $D_4$,
vitamin $D_5$,
vitamin $D_6$,
vitamin $D_7$,
24-oxovitamin $D_3$,
1α-hydroxyvitamin $D_2$,
1α-hydroxyvitamin $D_3$,
1α-hydroxyvitamin $D_4$,
1α-hydroxyvitamin $D_5$,
1α-hydroxyvitamin $D_6$,
1α-hydroxyvitamin $D_7$,
1α-hydroxy-24-oxovitamin $D_3$,
1a,24-dihydroxyvitamin $D_3$,
2α-hydroxyvitamin $D_2$,
2α-hydroxyvitamin $D_3$,
2α-hydroxyvitamin $D_4$,
2α-hydroxyvitamin $D_5$,
α-hydroxyvitamin $D_6$,
2α-hydroxyvitamin $D_7$,
2α-hydroxy-24-oxovitamin $D_3$,
2α,24-dihydroxyvitamin $D_3$,
24-hydroxyvitamin $D_3$,
23-hydroxyvitamin $D_3$,
23-hydroxyvitamin $D_4$,
1α,23-dihydroxyvitamin $D_3$,
1α,23-dihydroxyvitamin $D_4$,
2α,23-dihydroxyvitamin $D_3$,
2α,23-dihydroxyvitamin $D_4$,
26-hydroxyvitamin $D_2$,
26-hydroxyvitamin $D_3$,
26-hydroxyvitamin $D_4$,
1α,26-dihydroxyvitamin $D_2$,
1α,26-dihydroxyvitamin $D_3$,
1α,26-dihydroxyvitamin $D_4$,
2α,26-dihydroxyvitamin $D_2$,
2α,26-dihydroxyvitamin $D_3$,
2α,26-dihydroxyvitamin $D_4$,
23,24-dihydroxyvitamin $D_3$,
23,26-dihydroxyvitamin $D_3$,
23,26-dihydroxyvitamin $D_4$,
vitamin $D_3$-26,23-lactone,
1α-hydroxyvitamin $D_3$-26,23-lactone,
2α-hydroxyvitamin $D_3$-26,23-lactone,

24,24-difluorovitamin $D_3$,
24,24-dichlorovitamin $D_3$,
24,24-dibromovitamin $D_3$,
26,26,26,27,27,27-hexafluorovitamin $D_3$,
26,26,26,27,27,27-hexachlorovitamin $D_3$,
24,24-difluoro-25-hydroxy-26,27-dimethylvitamin $D_3$,
24,24-dichloro-25-hydroxy-26,27-dimethylvitamin $D_3$,
25-hydroxyvitamin $D_2$,
25-hydroxyvitamin $D_3$,
25-hydroxyvitamin $D_4$,
25-hydroxyvitamin $D_5$,
25-hydroxyvitamin $D_6$,
25-hydroxyvitamin $D_7$,
24-oxo-25-hydroxyvitamin $D_3$,
24,25-dihydroxyvitamin $D_3$,
23,25-dihydroxyvitamin $D_3$,
23,25-dihydroxyvitamin $D_4$,
25,26-dihydroxyvitamin $D_2$,
25,26-dihydroxyvitamin $D_3$,
25,26-dihydroxyvitamin $D_4$,
25-hydroxyvitamin $D_3$-26,23-lactone,
(10E)-19-fluorovitamin $D_3$,
(10E)-19-fluoro-1α-hydroxyvitamin $D_3$,
(10E)-19-fluoro-2α-hydroxyvitamin $D_3$,
4,4-difluorovitamin $D_3$,
4,4-difluoro-la-hydroxyvitamin $D_3$,
4,4-difluoro-2a-hydroxyvitamin $D_3$,
4,4-difluoro-provitamin $D_3$,
(10E)-19-chlorovitamin $D_3$,
(10E)-19-chloro-1α-hydroxyvitamin $D_3$,
(10E)-19-chloro-2a-hydroxyvitamin $D_3$,
4,4-dichlorovitamin $D_3$,
4,4-dichloro-1α-hydroxyvitamin $D_3$,
4,4-dichloro-2α-hydroxyvitamin $D_3$,
4,4-dichloro-provitamin $D_3$, and the like.

Examples of preferred compounds among these include vitamin $D_3$ derivatives represented by the following formula (II)

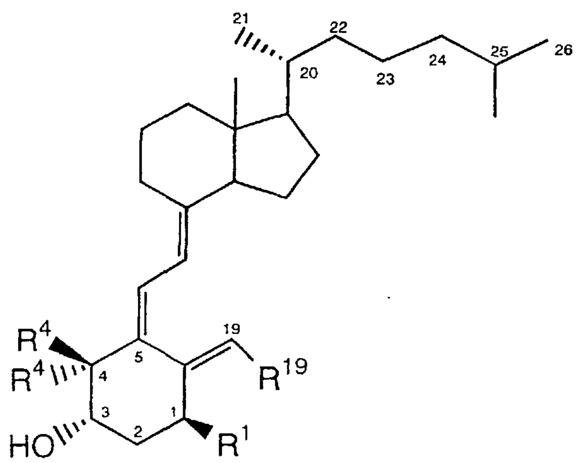

(II)

wherein the symbols have the same meanings as described above, specifically, vitamin $D_3$, 19-fluorovitamin $D_3$,

19-fluoro-1α-hydroxyvitamin $D_3$, 4,4-difluorovitamin $D_3$, 4,4-difluoro-1α-hydroxyvitamin $D_3$, 1α-hydroxyvitamin $D_3$ and the like.

The microorganisms capable of hydroxylating vitamin Ds, which are used in the present invention, belong to the genus *Nocardia*, *Streptomyces, Sphingomonas* or *Amycolata.*

Examples of those belonging to the genus *Nocardia* include *Nocardia autotrophica* N-102 (FERM BP-1573), etc.

Examples of those belonging to the genus *Streptomyces* include *Streptomyces roseosporus* A-5797 (FERM BP-1574), *Streptomyces sclerotialus* T-JSI (FERM BP-1370), etc.

Examples of those belonging to the genus *Sphingomonas* include *Sphingomonas mali* IFO15500, *Sphingomonas paucimobilis* IFO13935, *Sphingomonas parapaucimobilis* IFO15100, *Sphingomonas yanoikuyae* IFO15102, *Sphingomonas adhaesiva* IFO15099, *Sphingomonas capsulata* IFO12533, *Sphingomonas sanguis* IFO13937, *Sphingomonas macrogoltabidus* IFO15033, *Sphingomonas terrae* IFO15098.

Examples of those belonging to the genus Amycolata include *Amycolata saturnea* A-7983 (FERM BP-2307), *Amycolata saturnea* A-1246 (FERM BP-5544) as well as *Amycolata saturnea* IFO14499, *Amycolata autotrophica* ATCC19727, *Amycolata autotrophica* ATCC13181, *Amycolata autotrophica* ATCC33794, *Amycolata autotrophica* ATCC33795, *Amycolata autotrophica* ATCC33796, *Amycolata autotrophica* ATCC33797, *Amycolata autotrophica* JCM4010, *Amycolata hydrocarbonoxidans* IFO14498, and the like.

These may be used singly or in admixture of two or more thereof.

In the present invention, the hydroxylation reaction proceeds even without addition of a cyclodextrin, but preferably, the addition of a cyclodextrin is an effective procedure.

Cyclodextrins used herein are α-cyclodextrin, β-cyclodextrin, γ-cyclodextrin, β-dimethylcyclodextrin, β-trimethylcyclodextrin, partial methylated cyclodextrin (sometimes abbreviated as PMCD, hereinafter), branched cyclodextrin, and the like. These cyclodextrins may be used singly or in admixture of two or more thereof.

[Process of producing the compounds of the invention]

In the process of the present invention, the reaction rate and reaction efficiency can be enhanced by coexistence of cyclodextrin in the solution containing the microorganism or the enzyme produced by that microorganism. Liquid media are principally used as the media for culture of microorganisms, and glucose, maltose, sucrose, dextrin, starch, arabinose, xylose, glyceride, plant fats and oils, and animal fats and oils are used as a carbon source singly or in admixture. Soy bean powder, cottonseed meal, gluten meal, casein, peptone, casamino acids, yeast extract, meat extract, corn steep liquor and the like are used as a nitrogen source singly or in admixture. Other organic and inorganic materials which are needed for growth or facilitation of the production of hydroxylase for the 24-position, 25-position, 26-position, 1-position or 2-position can be added, if necessary.

An aerobic culture such as shaking culture and aerobic culture with stirring is suitable for the culture method.

The culture temperature is from 20 to 33°C, preferably from 25 to 30°C, and the vitamin D derivative as a substrate and if desired, the cyclodextrin, are added 12 to 96 hours, preferably 24 to 48 hours after the initiation of the culture. A suitable amount of the vitamin D derivative added is from 10 to 1000 μg/mL, and preferably from 50 to 500 μg/mL, in the medium, and a suitable amount of the cyclodextrin added is from 0.1 to 15% by weight, preferably from 0.1 to 2% by weight. A suitable pH in the medium is from 5 to 9, preferably from 6 to 8.

The production process of the present invention can be suitably performed by shaking or aerobically stirring the medium or the solution containing the microorganism or the enzyme. The aerobic stirring can be conducted for 1 to 130 hours after the addition of the vitamin D derivative as the substrate and if desired, cyclodextrin. Increasing pressure in an incubator or reacting under an oxygen gas stream is also effective for obtaining aerobic conditions.

The method of adding cyclodextrin can be any of the following methods; the method where the cyclodextrin is mixed with vitamin D derivative as the substrate in advance; the method where vitamin D derivative is first added, followed by addition of the cyclodextrin, and the method where the cyclodextrin is first added, followed by addition of vitamin D derivative.

Methods of isolating the vitamin D derivatives produced by these reactions include, for example, extraction with organic solvents, column chromatography, and the like. For example, after completion of the reactions, the reaction solution is extracted with methylene chloride, concentrated and dried up. This is dissolved in an appropriate solvent such as 2-propanol, n-hexane, and the like, insoluble matters are removed by filtration, centrifugation and the like, and then, the hydroxyvitamin D derivative can be isolated by high-performance liquid chromatography (HPLC) using a silica gel column, for example, Zorbax SIL (made by Du Pont, U.S.A.), Simpack ODS (made by Shimadzu Corporation), Hibar RT 250-10 LiChrosorb Si60 (made by Sica-Merck).

INDUSTRIAL APPLICABILITY

According to the process of the present invention, novel vitamin $D_3$ derivatives in which the hydrogen atom or atoms at the 2-position, 24-position, 25-position and/or 26-position are converted into a hydroxyl group or groups can be produced using a microorganism or an enzyme produced by that microorganism.
In particular, according to the process of the present invention, monohydroxylated or dihydroxylated compounds in which one or two of hydrogen atoms of corresponding substrate bound at the 2-position, 24-position, 25-positin and/or 26-position are converted into a hydroxyl group or groups can be readily produced.
The novel vitamin $D_3$ derivatives produced by the process of the present invention are expected to enhance their original activities such as absorption of calcium, facilitation of bone calcium metabolism, differentiation of epidermal keratinocytes and the like, to augment selectivity or to improve *in vivo* absorptivity.

[Pharmacological activity]

Evaluation studies on (1) binding ability to vitamin D receptor (VDR), (2) binding ability to vitamin D-bound protein (DBP) and (3) induction ability of differentiation of HL-60 were performed on the following eight novel vitamin $D_3$ derivatives described in the Examples and a control compound (specimens 1 to 9).

Specimen 1: Control (1α,25-dihydroxyvitamin $D_3$ ($1,25(OH)_2D_3$))
Specimen 2: Example 1(1) (2α,25-dihydroxyvitamin $D_3$)
Specimen 3: Example 2 (1α,26-dihydroxyvitamin $D_3$)
Specimen 4: Example 4(1) (19-fluoro-1α,24-dihydroxyvitamin $D_3$)
Specimen 5: Example 4(2) (19-fluoro-1α,25-dihydroxyvitamin $D_3$)
Specimen 6: Example 4(3) (19-fluoro-1α,26-dihydroxyvitamin $D_3$)
Specimen 7: Example 5(1) (4,4-difluoro-24-hydroxyvitamin $D_3$)
Specimen 8: Example 5(3) (4,4-difluoro-26-dihydroxyvitamin $D_3$)
Specimen 9: Example 6(2) (4,4-difluoro-1α,25-dihydroxyvitamin $D_3$)

(1) Binding ability to vitamin D receptor (VDR):

Binding ability to VDR was examined by the following radio receptor assay (RRA) method.
$1,25(OH)_2D_3$ and each specimen were serially diluted at from $10^{-11}$ M to $10^{-6}$ M. 20μL of a [26,27-$^3$H]-1,25-dihydroxyvitamin $D_3$ ($^3H$-$1,25(OH)_2D_3$) solution and 60 μL of $1,25(OH)_2D_3$ or each specimen solution were added in a small test tube, and 0.5 mL of a VDR (chicken small intestine receptor) solution was added and mixed, and then allowed to stand at 4°C for 3 hours. Then, after 0.125 mL of a dextran-active carbon suspension was added and mixed, the mixture was allowed to stand on ice for 30 minutes, $^3H$-$1,25(OH)_2D_3$ unbound to VDR was removed by centrifugation at 3000 rpm for 15 minutes, and then the quantity of $^3H$-1,25 $(OH)_2D_3$ bound to VDR was measured using 0.5 mL of the supernatant.
The binding ability to VDR of each specimen was evaluated by calculating and comparing the $B/B_0$ 50% value. This is the amount of the specimen bound to VDR when the amount (B) of $^3H$-$1,25(OH)_2D_3$ bound to VDR was competitively inhibited by the specimen to 50% of $B_0$, which is the amount of $^3H$-$1,25(OH)_2D_3$ bound to VDR when only $^3H$-$1,25(OH)_2D_3$ exists. The VDR binding ability was shown in Table 1 as a relative value of each specimen when $B/B_0$ 50% value of $1,25(OH)_2D_3$ is taken to be 100.
Various actions of the vitamin D such as regulation of calcium metabolism, suppression of cellular proliferation, induction of differentiation, and the like are expressed via the vitamin D receptor (VDR). As shown in Table 1, the vitamin $D_3$ derivatives of the present invention have the binding ability to VDR and the medicinal use that possesses various actions as described above is expected.

(2) Binding ability to vitamin D-bound protein (DBP):

The binding ability to DBP was evaluated using the following competitive protein binding assay (CPBA). 1,25 $(OH)_2D_3$ and each specimen were diluted to concentrations from $10^{-11}$ M to $10^{-5}$ M. 50 μL of a [26,27-$^3$H]-25-hydroxyvitamin $D_3$ ($^3H$-$25(OH)D_3$) solution and 100 μL of $1,25(OH)_2D_3$ or each specimen solution were placed in a small test tube, and 1 mL of a DBP (rat serum diluted with a barbital acetate buffer) solution was added and mixed, and then allowed to stand at 4°C for 1 hour. Then, after 0.5 mL of a dextran-active carbon suspension was added and mixed, the mixture was allowed to stand on ice for 10 minutes, $^3H$-$25(OH)D_3$ unbound to DBP was removed by centrifugation at 3000 rpm for 15 minutes, and then the quantity of $^3H$-$25(OH)D_3$ bound to DBP was measured using 1 mL of the supernatant.

The binding ability to DBP of each specimen was evaluated by calculating and comparing the $B/B_0$ 50% value. This is the amount of the specimen bound to DBP when the amount (B) of $^3H$-25(OH)$D_3$ bound to DBP was competitively inhibited by the coexisting specimen and 1,25$(OH)_2D_3$ to 50% of $B_0$, which is the amount of $^3H$-25(OH)$D_3$ bound to DBP when only $^3H$-25(OH)$D_3$ exists. The DBP binding ability was shown in Table 1 as a relative value of each specimen when $B/B_0$ 50% value of 1,25$(OH)_2D_3$ is taken to be 100.

Vitamin Ds are stable in blood when they are bound to the vitamin D binding protein (DBP). The property of vitamin $D_3$ derivatives of the present invention to bind to DBP implies their stability in blood, suggesting that the vitamin D derivatives transported to sites and organs in needs via blood can express actions as vitamin D. Thus, the vitamin $D_3$ derivatives of the present invention are expected to be useful as medicines which exist stably in blood or are transported to target organs via a blood flow.

(3) Ability to induce differentiation of HL60:

HL60 cells were passaged in RPMI 1640 medium containing 10% fetal bovine serum in an atmosphere 5% $CO_2$ at 37°C.

Differentiation of HL60 cells by vitamin D derivative was induced by culturing in the media containing from $10^{-11}$ to 10 $M^{-5}$ of 1,25$(OH)_2D_3$ or each specimen at 37°C for 4 days. The ability to induce differentiation was evaluated by measuring reduction of cytochrome C as an index in respect of the amount of super oxide which was produced by the cells stimulated with phorbol myristate acetate (PMA). Namely, after removing the culture medium by aspiration, the cells treated with vitamin D derivatives were suspended in 1.5 mL of a reaction mixture (ferricytochrome C (80 μM), PMA (500 ng/mL)) and cultured at 37°C for 1 hour. After completion of the culture, an absorbance, $OD_{550}$~$OD_{540}$ of a centrifuged supernatant was measured. A concentration of the reduced cytochrome C was calculated by the following formula using a molecular absorption constant, $19.1 \times 10^3$ $cm^{-1}$.

$$\text{Concentration of reduced cytochrome C (nmol/L)} = (OD_{550}\text{-}OD_{540})/19.1 \times 10^3 \times 1.5/10^3 \times 10^9$$

The induction ability of differentiation of HL-60 was compared with respect to the $EC_{50}$ value calculated from the concentration of reduced cytochrome C, and a relative value of each specimen is shown in Table 1 when the $EC_{50}$ value of 1,25$(OH)_2D_3$ is taken to be 100.

The vitamin Ds have the action that leads undifferentiated tumor cells to differentiated normal cells (macrophages). The HL-60 cells are a human promyelocytic leukemia cell strain, and as shown in Table 1, the vitamin $D_3$ derivatives of the present invention having the action to induce HL-60 differentiation are expected to possess medicinal use that induces differentiation.

Table 1

| Specimen | | Binding ability to VDR | Induction ability of differentiation of HL-60 | Binding ability to DBP |
|---|---|---|---|---|
| No. | | | | |
| 1 | Control | 100.00 | 100.00 | 100.00 |
| 2 | Example 1(1) | 32.480 | 3.285 | 11433.718 |
| 3 | Example 2 | 107.557 | 34.715 | 168.529 |
| 4 | Example 4(1) | 72.878 | 196.572 | 23.665 |
| 5 | Example 4(2) | 62.386 | 87.404 | 35.792 |
| 6 | Example 4(3) | 29.129 | 12.296 | 35.257 |
| 7 | Example 5(1) | 81.860 | 105.243 | 16.633 |
| 8 | Example 5(3) | 34.067 | <6.092 | 28.106 |
| 9 | Example 6(2) | 92.716 | 79.906 | 26.684 |

BEST MODE FOR CARRYING OUT THE INVENTION

The present invention will be described in details by the following Examples, but it should not be construed that the invention is limited to these Examples. The percentages below are on a weight basis unless otherwise designated.

The following methods were used for instrumental analysis of each sample in the Examples.

A sample after microbial conversion was isolated and purified using an HPLC system equipped with an MD-910 type multiple detector supplied by JASCO Corporation, and then subjected to the following analyses.
$^{1}$H-NMR and $^{19}$F-NMR were measured by Type ARX-400 supplied by Bruker. Tetramethylsilane (TMS) as an internal standard for $^{1}$H-NMR and trifluorotoluene as an external standard ($\delta$ = -63 ppm) for $^{19}$F-NMR were used, and chemical shifts were represented as $\delta$ values. NMR data were described by the following abbreviations ; s = singlet, d = doublet, t = triplet, m = multiplet, and br = broad signal. MS spectra were measured by an electron impact (EI) method using Type JMS-AX505HA supplied by JEOL Ltd. UV spectra were measured using Type U-3200 supplied by Hitachi, Ltd.

Example 1: Microbial conversion of vitamin $D_3$

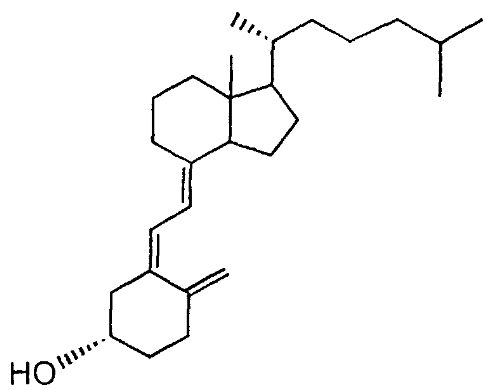

100 mL of a medium (BG medium) consisting of 1.5% bactosoyton (supplied by Difco), 0.5% corn steep liquor, 1.5% glucose, 0.5% NaCl, and 0.2% $CaCO_3$ (pH 7.2) was placed in a 500 mL-Erlenmeyer flask and autoclaved at 120°C for 20 minutes. This was inoculated with one platinum loop of *Amycolata autotrophica* ATCC 33797 and shaken at 28°C for 72 hours to culture aerobically.
The cultured broth was centrifuged, and the mycelium were collected and washed once with 0.01 M of tris-acetate buffer (containing 2 mM magnesium acetate, 7 mM 2-mercaptoethanol and 20% glycerin) (pH 7.4), , followed by suspension in 100 mL of the same buffer. This suspension was sonicated (at 20 kHz and 50 W for 2 minutes) and centrifuged (10,000 × g for 15 minutes ) to obtain the supernatant. Polyethylene Glycol 6000 was gradually added to this supernatant to a final concentration of 25%, followed by allowing to stand at 4°C for 10 minutes. A crude enzyme precipitation was obtained by centrifugation. This crude enzyme precipitation at a wet weight of 1 g was suspended in 10 mL of tris-acetate buffer (containing 70 mM nicotine amide, 2 mM magnesium acetate, 100 mM NADP, 5 mM ATP, and 6 mM glucose-6-phosphate)(pH 7.4), followed by adding 5 units of glucose-6-phosphate dehydrogenase, 0.2 mL of a solution of 10 mg/mL vitamin $D_3$ in ethanol and 0.1 mL of a 50% partial methylated β-cyclodextrin (supplied by Mercian Corporation) solution thereto. Then, the mixture was stirred and shaken at 28°C for one hour to effect enzymatic reaction.
After completion of the reaction, methanol at the equivalent amount to the cultured solution was added and mixed, then centrifuged at 3000 rpm for 10 minutes (Type CST060LF type, supplied by Shimadzu Corporation) to collect the supernatant. The collected supernatant was passed through a column (an inner diameter 1 cm × a length 15 cm) packed with Diaion HP20SS (supplied by Mitsubishi Chemical Industries, Ltd.) at a flow rate of 0.5 mL/min., and eluted with a methanol concentration gradient from 50 to 100% to obtain a peak fraction. This peak fraction was concentrated, dried up, and dissolved in 3 mL of a mixed solvent of 2-propanol/n-hexane (1/9), then allowed to stand at -20°C for 2 hours. The resulting insoluble component was eliminated by centrifugation. This supernatant was concentrated under reduced pressure to afford a product (2.2 mg). Each peak fraction was collected by HPLC and concentrated under reduced pressure and dried up while purging with a nitrogen gas at 40°C or less to afford 2α,25-dihydroxyvitamin $D_3$ (20 µg) as shown below.
Also, fractions other than the above fraction were purified by semi-preparative HPLC to afford 1α,25-dihydroxyvitamin $D_3$ (400 µg).
HPLC conditions:

Column: Hibar RT 250-10 LiChrosorb Si60 (7 µm)

Elution solvent: 15% 2-propanol-hexane
Flow rate: 7 mL/min.
Temperature: room temperature
Detection wavelength: 265 nm

(1) 2α,25-Dihydroxyvitamin $D_3$ (novel compound):

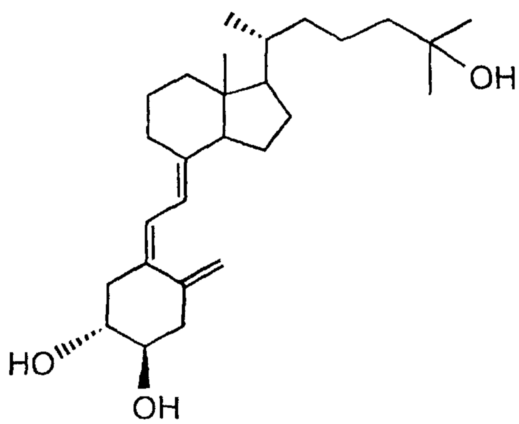

$^1$H-NMR ($CDCl_3$) : δ 0.54 (3H, s, 18-H), 0.93 (3H, d, J=6.4 Hz, 21-H), 1.22 (6H, s, 26-H, 27-H), 2.62 (2H, m, 1-H, 4-H), 2.82 (1H, m, 9-H), 3.59 (2H, m, 2-H, 3-H), 4.91 (1H, dd, J=1.8, 1.8 Hz, 19-H), 5.12 (1H, br.s, 19-H), 5.9 (1H, d, J=11.3 Hz, 7-H), 6.27 (1H, d, J=11.3 Hz, 6-H);
$^{13}$C-NMR ($CDCl_3$) : δ 12.3, 19.0, 21.0, 22.3, 23.7, 27.9, 29.2, 29.4, 29.5, 36.3, 36.6, 40.7, 41.2, 42.2, 44.6, 46.1, 56.5, 56.7, 71.4, 74.8, 75.1, 115.2, 117.6, 122.9, 134.2, 141.9, 143.3;
MSm/z (%): 416 ($M^+$, 49), 398(21), 383(21), 365(14), 287(17), 269(10), 251(9), 152(100), 134(49);
UV $\lambda_{max}$ (EtOH): 265 nm

(2) 1α,25-Dihydroxyvitamin $D_3$:

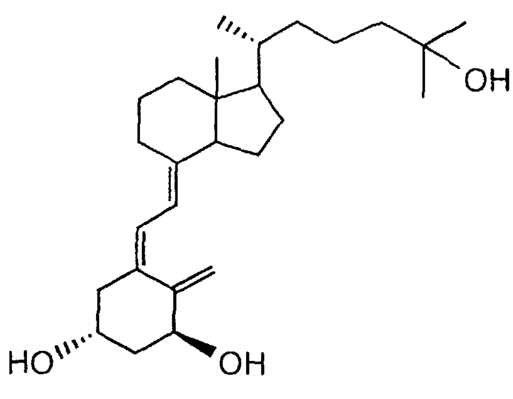

$^1$H-NMR ($CDCl_3$) : δ 0.54 (3H, s, 18-H), 0.94 (3H, d, J=6.4 Hz, 21-H), 1.22 (6H, s, 26-H, 27-H), 2.31 (1H, dd, J=13.3, 6.6 Hz, 4-H), 2.60 (1H, dd, J=13.3, 3.3 Hz, 4-H), 2.82 (1H, m, 9-H), 4.23 (1H, m, 3-H), 4.43 (1H, m, 1-H), 5.01 (1H, s, 19-H), 5.33 (1H, s, 19-H), 6.02 (1H, d, J=11.2 Hz, 7-H), 6.38 (1H, d, J=11.2 Hz, 6H);
MSm/z (%): 416($M^+$, 15), 398(91), 380(93), 365(18), 362(24), 287(9), 285(16), 251(27), 152(36), 134(100);
UV $\lambda_{max}$ (EtOH): 264 nm

Example 2: Microbial conversion of 1α-hydroxyvitamin $D_3$

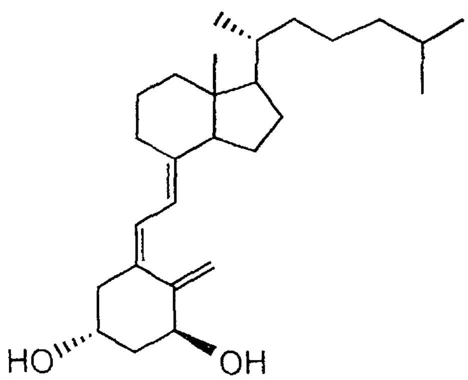

100 mL of a medium (BG medium) consisting of 1.5% bactosoyton (supplied by Difco), 0.5% corn steep liquor, 1.5% glucose, 0.5% NaCl, and 0.2% $CaCO_3$ (pH 7.2) was placed in a 500 mL-Erlenmeyer flask and autoclaved at 120°C for 20 minutes. This was inoculated with one platinum loop of *Amycolata autotrophica* ATCC 33797 and shaken at 28°C and at 220 rpm for 48 hours to culture aerobically.

Also, 50 mL of a BG medium having 1% partial methylated β-cyclodextrin (supplied by Mercian Corporation) added thereto was placed in a 250 mL-Erlenmeyer flask and autoclaved at 121°C for 20 minutes.

To this BG medium, 1 mL of the previously cultured broth was transferred sterilely, and cultured at 28°C and at 200 rpm for 48 hours. To this culture solution, 1α-hydroxyvitamin $D_3$ (15 mg) dissolved in 0.5 mL of ethanol was added and subjected to enzymatic reaction for 33 hours.

After completion of the reaction, methanol at the equivalent amount to the cultured solution was added and mixed, then centrifuged at 3000 rpm for 10 minutes (Type CST060LF, supplied by Shimadzu Corporation) to collect the supernatant. The collected supernatant was passed through a column (an inner diameter 1 cm × a length 15 cm) packed with Diaion HP20SS (supplied by Mitsubishi Chemical Industries, Ltd.) at a flow rate of 0.5 mL/min. and eluted with a methanol concentration gradient from 50 to 100% to obtain a peak fraction. This peak fraction was concentrated, dried up, and dissolved in 3 mL of a mixed solvent of 2-propanol/n-hexane (1/9), then allowed to stand at -20°C for 2 hours. The resulting insoluble component was eliminated by centrifugation. This supernatant was concentrated under reduced pressure to afford a product (20.5 mg). Each peak fraction was collected by HPLC and concentrated under reduced pressure and dried up while purging with a nitrogen gas at 40°C or lower to afford ,1α 24-dihydroxyvitamin $D_3$ (808 μg), 1α,25-dihydroxyvitamin $D_3$ (5.41 mg) and 1α,26-dihydroxyvitamin $D_3$ (531 μg).
HPLC conditions:

Column: Hibar RT 250-10 LiChrosorb Si60 (7 μm)
Elution solvent: 15% 2-propanol-hexane
Flow rate: 7 mL/min.
Temperature: room temperature
Detection wavelength: 265 nm

Spectral data: 1α,26-Dihydroxyvitamin $D_3$ (novel compound)

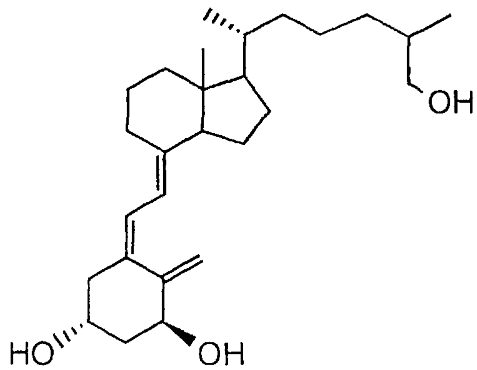

$^1$ H-NMR ($CDCl_3$) : δ 0.54 (3H, s, 18-H), 0.92 and 0.93 (each 3H, d, J=6.6 Hz, 21-H, 27-H), 2.31 (1H, dd, J=13.3, 6.6 Hz, 4-H), 2.60 (1H, dd, J=13.3, 3.2 Hz, 4-H), 2.82 (1H, m, 9-H), 3.42 (1H, dd, J=10.5, 6.5 Hz, 26-H), 3.52 (1H, dd, J=10.5, 5.6 Hz, 26-H), 4.23 (1H, m, 3-H), 4.43 (1H, m, 1-H), 5.01 and 5.33 (each 1H, s, 19-H), 6.01 (1H, d, J=11.3 Hz, 7-H), 6.38 (1H, d, J=11.3 Hz, 6-H);
MS m/z (%): 416($M^+$, 38), 398(58), 380(48), 365(5), 287(9), 251(16), 152(50), 134(100);
UV $\lambda_{max}$ (EtOH) : 264 nm

Example 3: Microbial conversion of 19-fluorovitamin $D_3$

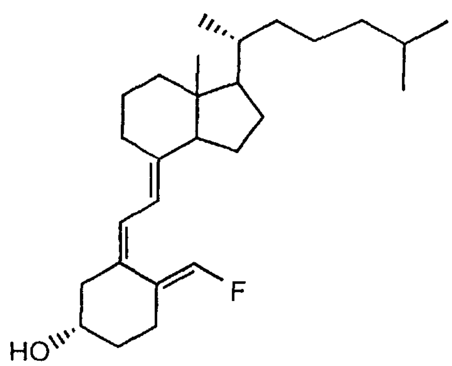

The same procedure as in Example 2 was carried out to obtain a product (1.7 mg), except that 19-fluorovitamin $D_3$ (4.90 mg) was used as the substrate. After purification by semi-preparative HPLC under the following condition, 19-fluoro-24-hydroxyvitamin $D_3$ (14 μg), 19-fluoro-25-hydroxyvitamin $D_3$ (206 μg) and 19-fluoro-26-hydroxyvitamin $D_3$ (28 μg) were obtained.
HPLC conditions:

Column: Hibar RT 250-10 LiChrosorb Si60 (7 μm)
Elution solvent: 8% 2-propanol-hexane
Flow rate: 5 mL/min.
Temperature: room temperature
Detection wavelength: 265 nm

Spectral data:

(1) 19-Fluoro-24-hydroxyvitamin $D_3$ (novel compound):

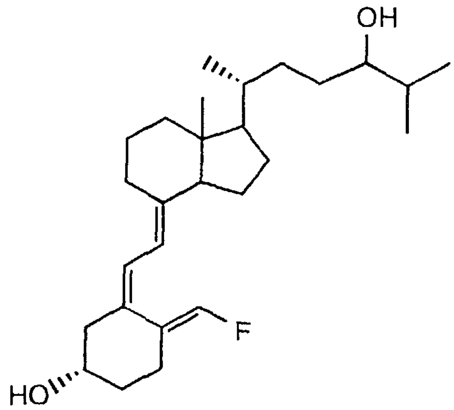

$^1$H-NMR ($CDCl_3$): δ 0.55 (3H, s, 18-H), 0.90-0.95 (m, 21-H, 26-H, 27-H), 2.57 (2H, m), 2.80 (1H, m, 9-H), 3.33 (1H, m, 24-H), 3.93 (1H, m, 3-H), 5.93 (1H, d, J=11.2 Hz, 7-H), 6.28 (11H, d, J=11.1 Hz, 6-H), 6.51 (1H, d, J=87.3 Hz, 19-H);
MS m/z (%): 418($M^+$, 28), 400(19), 398(21), 380(21), 362(6), 289(15), 271(19), 269(16), 251(17), 154(57), 136 (98), 135(100);
UV $\lambda_{max}$ (EtOH): 261.2 nm

(2) 19-Fluoro-25-hydroxyvitamin $D_3$:

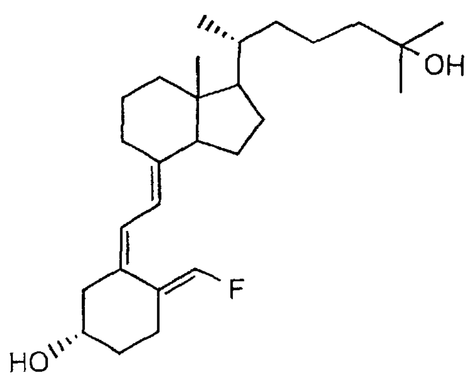

$^1$H-NMR ($CDCl_3$) : δ 0.54 (3H, s, 18-H), 0.94 (3H, d, J=6.4 Hz, 21-H), 1.22 (6H, s, 26-H, 27-H), 2.26 (1H, dd, J=13.1, 7.8 Hz), 2.56 (2H, m), 2.79 (1H, m, 9-H), 3.94 (1H, m, 3-H), 5.93 (1H, d, J=11.1 Hz, 7-H), 6.28 (1H, d, J=11.1 Hz, 6-H), 6.51 (1H, d, J=87.3 Hz, 19H);
$^{19}$F-NMR ($CDCl_3$) δ: -132.5(d, J=87.3 Hz);
MS m/z (%): 418($M^+$, 7), 400(18), 380(13), 362(6), 289(9), 271(10), 269(10), 251(13), 154(53), 136(100), 135(98);
UV $\lambda_{max}$ (EtOH): 260.0 nm

(3) 19-Fluoro-26-hydroxyvitamin $D_3$ (novel compound):

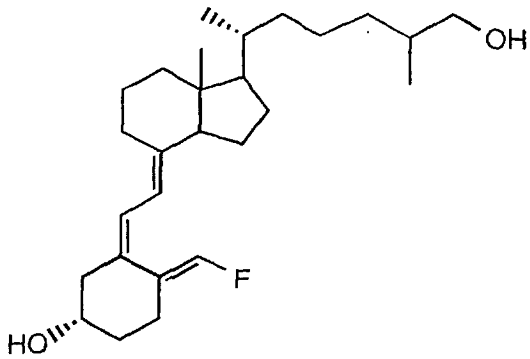

$^1$H-NMR ($CDCl_3$) : δ 0.54 (3H, s, 18-H), 0.90-0.94 (6H, m, 21-H, 27-H), 2.80 (1H, m, 9-H), 3.40-3.56 (2H, m, 26-H), 3.94 (1H, m, 3-H), 5.93 (1H, d, J=11.1 Hz, 7-H), 6.28 (1H, d, J=11.1 Hz, 6-H), 6.51 (1H, d, J=87.3 Hz, 19H);
MS m/z (%): 418 ($M^+$, 18), 400(40), 380(16), 362(9), 289(27), 271(21), 269(12), 251(16), 154(55), 136(91), 135 (100);
UV $\lambda_{max}$ (EtOH): 260 nm

Example 4: Microbial conversion of 19-fluoro-1α-hydroxyvitamin $D_3$

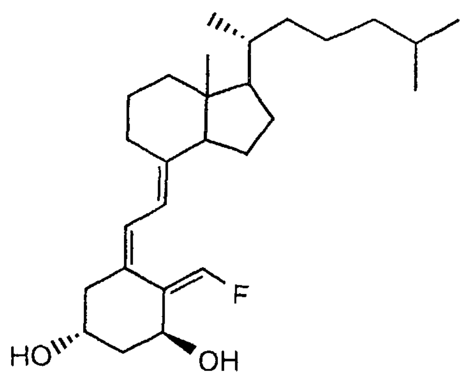

The same procedure as in Example 2 was carried out to obtain a product (2.8 mg), except that 19-fluoro-1α-hydroxyvitamin $D_3$ (2.44 mg) was used as the substrate. After purification by semi-preparative HPLC under the following condition, 19-fluoro-1α,24-dihydroxyvitamin $D_3$ (46 μg), 19-fluoro-1α,25-dihydroxyvitamin $D_3$ (980 μg) and 19-fluoro-1α,26-dihydroxyvitamin $D_3$ (50 μg) were obtained.
HPLC conditions:

Column: Hibar RT 250-10 LiChrosorb Si60 (7 μm)
Elution solvent: 15% 2-propanol-hexane
Flow rate: 6 mL/min.
Temperature: room temperature
Detection wavelength: 265 nm

Spectral data:

(1) 19-Fluoro-1α,24-dihydroxyvitamin $D_3$ (novel compound):

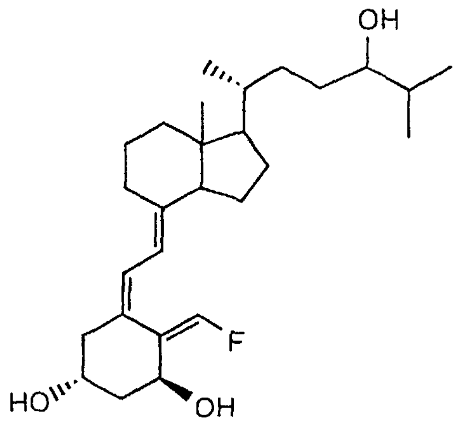

$^1$H-NMR ($CDCl_3$): δ 0.54 (3H, s, 18-H), 0.917 and 0.927 (each 3H, d, J=6.9 Hz, 26-H, 27-H), 0.935 (3H, d, J=6.7 Hz, 21-H), 2.19 (1H, m), 2.32 (1H, m), 2.68 (1H, m), 2.80 (1H, m, 9-H), 3.32 (1H, m, 24-H), 4.17 (1H, m, 3-H), 5.09 (1H, m, 1-H), 5.90 (1H, d, J=11.2 Hz, 7-H), 6.46 (1H, d, J=11.2 Hz, 6-H), 6.50 (1H, d, J=86.0 Hz, 19-H);
MS m/z (%): 434($M^+$, 8), 416(4), 396(9), 378(28), 360(100), 135(40);
UV $\lambda_{max}$ (EtOH): 261.6 nm

(2) 19-Fluoro-1α,25-dihydroxyvitamin $D_3$:

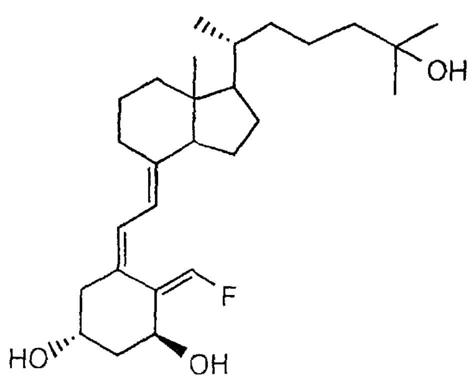

$^1$H-NMR ($CDCl_3$) : δ 0.53 (3H, s, 18-H), 0.94(3H, d, J=6.4 Hz, 21-H), 1.22 (6H, s, 26-H, 27-H), 2.19 (1H, t, J=11.5 Hz, 4-H), 2.32 (1H, m), 2.68 (1H, m), 2.80 (1H, m, 9-H), 4.17 (1H, m, 3-H), 5.09 (1H, m, 1-H), 5.90 (1H, d, J=11.1 Hz, 7-H), 6.47 (1H, d, J=11.1 Hz, 6-H), 6.51 (1H, d, J=86.1 Hz, 19H);
$^{19}$F-NMR ($CDCl_3$): δ -129.8 (d, J=86.1 Hz);
MS m/z (%): 434($M^+$, 2), 416(9), 396(9), 378(17), 360(100), 305(2), 287(4), 267(6), 135(32);
UV $\lambda_{max}$ (EtOH): 262.2 nm

(3) 19-Fluoro-1α,26-dihydroxyvitamin $D_3$ (novel compound):

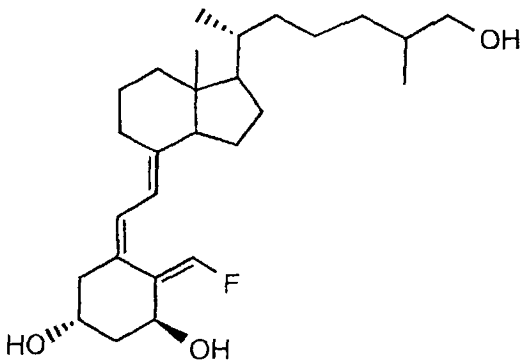

$^1$H-NMR ($CDCl_3$): δ 0.53 (3H, s, 18-H), 0.92 and 0.93 (each 3H, d, J=6.2 Hz, 21-H, 27-H), 2.19 (1H, m), 2.32 (1H, m), 2.68 (1H, m, 4-H), 2.80 (1H, m, 9-H), 3.40-3.56 (2H, m, 26-H), 4.17 (1H, m, 3-H), 5.09 (1H, m, 1-H), 5.90 (1H, d, J=11.7 Hz, 7-H), 6.46 (1H, d, J=11.7 Hz, 6-H), 6.51 (1H, d, J=86.0 Hz, 19H);
MS m/z (%): 434($M^+$, 9), 416(8), 396(17), 378(56), 360(21), 305(3), 287(10), 267(12), 135(100);
UV $\lambda_{max}$ (EtOH): 263.2 nm

Example 5: Microbial conversion of 4,4-difluorovitamin $D_3$

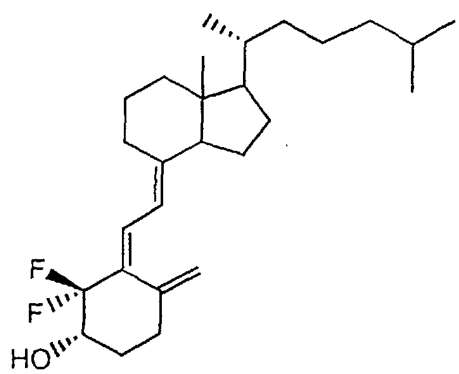

The same procedure as in Example 2 was carried out to obtain a product (3.3 mg), except that 4,4-difluorovitamin $D_3$ (4.90 mg) was used as the substrate. After purification by semi-preparative HPLC under the following condition, 4,4-difluoro-24-hydroxyvitamin $D_3$ (86 μg), 4,4-difluoro-25-hydroxyvitamin $D_3$ (857 μg) and 4,4-difluoro-26-hydroxyvitamin $D_3$ (137 μg).
HPLC conditions:

Column: Hibar RT 250-10 LiChrosorb Si60 (7 μm)
Elution solvent: 7% 2-propanol-hexane
Flow rate: 5 mL/min.
Temperature: room temperature
Detection wavelength: 265 nm

Spectral data:

(1) 4,4-Difluoro-24-hydroxyvitamin $D_3$ (novel compound):

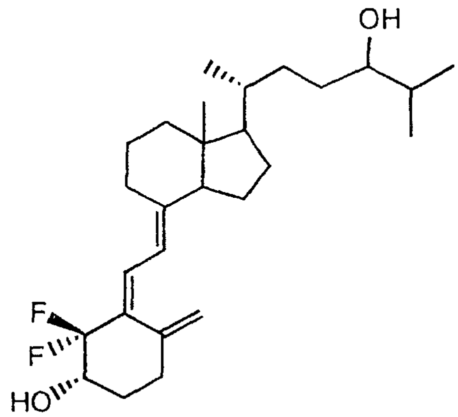

$^1$H-NMR ($CDCl_3$): δ 0.56 (3H, s, 18-H), 0.92 and 0.93 (each 3H, d, J=6.6 Hz, 26-H, 27-H, overlapped with 21-H), 2.47 (1H, m), 2.90 (1H, m, 9-H), 3.32 (1H, m, 24-H), 3.98 (1H, m, 3-H), 5.00 (1H, s, 19-H), 5.23 (1H, s, 19-H), 6.09 (1H, d, J=11.4 Hz, 7-H), 6.94 (1H, d, J=11.4 Hz, 6-H);
MS m/z (%): 436($M^+$, 34), 418(6), 398(83), 380(74), 378(48), 307(28), 269(100), 135(79);
UV $\lambda_{max}$ (EtOH): 269.8 nm

(2) 4,4-Difluoro-25-hydroxyvitamin $D_3$:

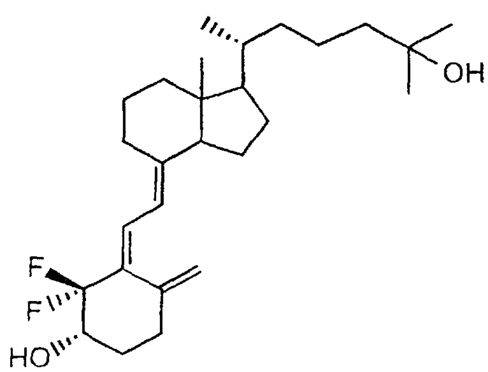

$^1$H-NMR ($CDCl_3$) : δ 0.55 (3H, s, 18-H), 0.94 (3H, d, J=6.4 Hz, 21-H), 1.22 (6H, s, 26, 27-H), 2.21 (1H, m), 2.47 (1H, m), 2.89 (1H, m, 9-H), 3.98 (1H, m, 3-H), 5.00 (1H, d, J=1.4 Hz, 19-H), 5.23 (1H, s, 19-H), 6.09 (1H, d, J=11.5 Hz, 7-H), 6.94 (1H, d, J=11.5 Hz, 6-H);
$^{19}$F-NMR ($CDCl_3$) : δ-114.1(dd, J=234.1 and 11.5 Hz), -108.0(d, J=234.1 Hz);
MS m/z (%) : 436($M^+$, 61), 418(61), 398(60), 380(100), 378(38), 307(64), 269(58), 135(79);
UV $\lambda_{max}$ (EtOH): 269.6 nm

(3) 4,4-Difluoro-26-hydroxyvitamin $D_3$ (novel compound):

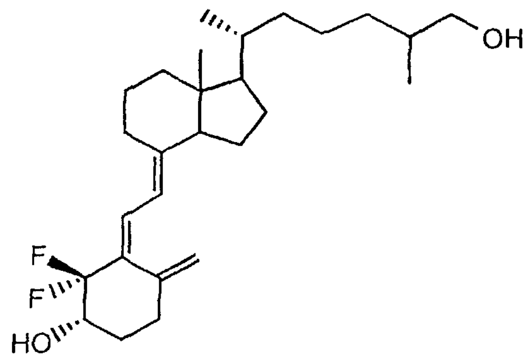

$^1$H-NMR ($CDCl_3$): δ 0.55 (3H, s, 18-H), 0.92-0.94 (m, 21-H, 27-H), 2.35 (1H, m), 2.47 (1H, m), 2.89 (1H, m, 9-H), 3.40-3.56 (2H, m, 26-H), 3.98 (H, m, 3-H), 5.00 (1H, d, J=1.4 Hz, 19-H), 5.23 (1H, s, 19-H), 6.09 (1H, d, J=11.5 Hz, 7-H), 6.94 (1H, d, J=11.5 Hz, 6-H);
MSm/z (%): 436($M^+$, 71), 418(10), 398(44), 378(34), 376(100), 307(48), 269(33), 135(70);
UV $\lambda_{max}$ (EtOH): 270.0 nm

Example 6: Microbial conversion of 4,4-difluoro-1α-hydroxy vitamin $D_3$:

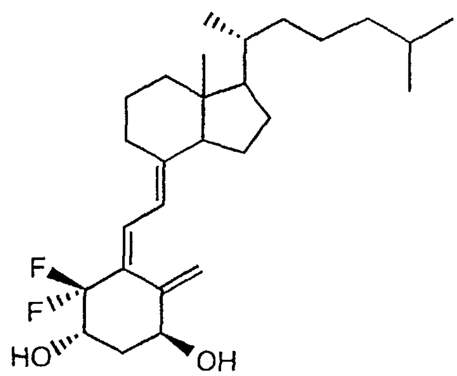

The same procedure as in Example 2 was carried out to obtain a product (0.9 mg), except that 4,4-difluoro-1α-hydroxyvitamin $D_3$ (1.76 mg) was used as the substrate. After purification by semi-preparative HPLC under the following condition, 4,4-difluoro-1α,24-dihydroxyvitamin $D_3$ (115 μg), 4,4-difluoro-1α,25-dihydroxyvitamin $D_3$ (652 μg), and 4,4-difluoro-1α,26-dihydroxyvitamin $D_3$ (33 μg) were obtained.
HPLC conditions:

Column: Hibar RT 250-10 LiChrosorb Si60 (7 μm)
Elution solvent: 15% 2-propanol-hexane
Flow rate: 6 mL/min.
Temperature: room temperature
Detection wavelength: 265 nm

Spectral data:

(1) 4,4-Difluoro-1α,24-dihydroxyvitamin $D_3$ (novel compound):

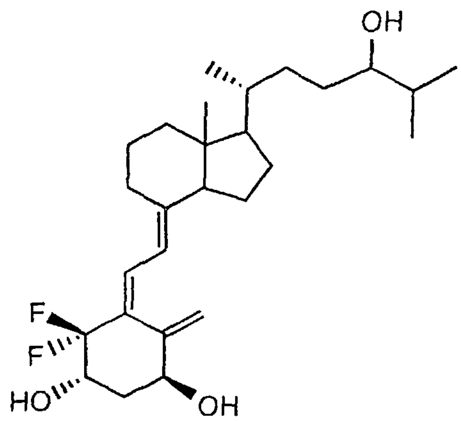

$^1$H-NMR ($CDCl_3$): δ 0.56 (3H, s, 18-H), 0.92 and 0.93 (each 3H, d, J=6.7 Hz, 26-H, 27-H, overlapped with 21-H), 2.90 (1H, m, 9-H), 3.32 (1H, m, 24-H), 4.26 (1H, m, 3-H), 4.49 (1H, m, 1-H), 5.18 (1H, s, 19-H), 5.50 (1H, s, 19-H), 6.07 (1H, d, J=11.4 Hz, 7-H), 7.06 (1H, d, J=11.4 Hz, 6-H);
MS m/z (%): 452($M^+$, 34), 434(25), 414(41), 396(100), 378(80), 376(80), 323(29), 305(33), 285(46), 267(79), 265 (54), 135(92);
UV $\lambda_{max}$ (EtOH): 270.6 nm.

(2) 4,4-Difluoro-1α,25-dihydroxyvitamin $D_3$:

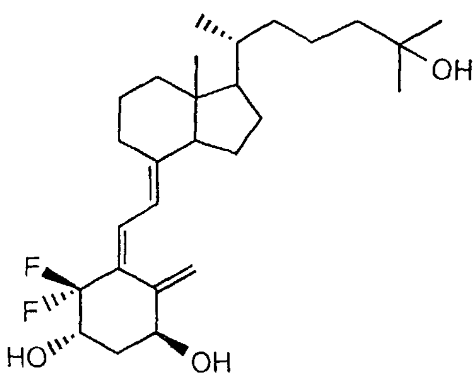

$^1$ H-NMR ($CDCl_3$): δ 0.56 (3H, s, 18-H), 0.94 (3H, d, J=6.3 Hz, 21-H), 1.22 (6H, s, 26-H, 27-H), 2.90 (1H, m, 9-H), 4.25 (1H, m, 3-H), 4.49 (1H, m, 1-H), 5.18 (1H, s, 19-H), 5.50 (1H, s, 19-H), 6.07 (1H, d, J=11.5 Hz, 7-H), 7.06 (1H, d, J=11.4 Hz, 6-H);
$^{19}$F-NMR ($CDCl_3$) : δ-116.3 (d, J=234.2 Hz), -107.7 (d, J=234.2 Hz);
MS m/z (%): 452($M^+$, 15), 434(45), 416(22), 396(65), 378(58), 376(31), 323(49), 305(20), 285(27), 267(48), 265 (27), 135(100);
UV $\lambda_{max}$ (EtOH): 270.6 nm

(3) 4,4-Difluoro-1α,26-dihydroxyvitamin $D_3$ (novel compound)

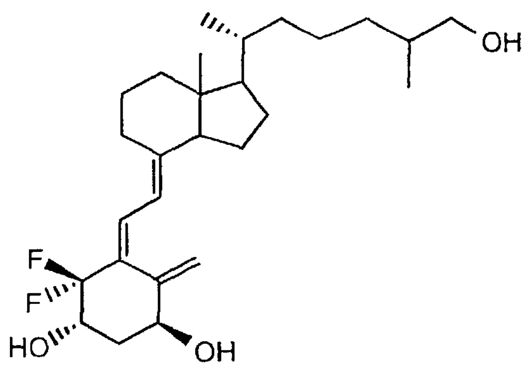

$^1$H-NMR ($CDCl_3$): δ 0.55(3H, s, 18-H), 0.92 and 0.93 (each 3H, d=6.8 Hz, 26-H, 27-H, overlapped with 21-H), 2.90 (1H, m, 9-H), 3.40-3.56 (2H, m, 26-H), 4.26 (1H, m, 3-H), 4.49 (1H, m, 1-H), 5.18 (1H, s, 19-H), 5.50 (1H, s, 19-H), 6.07 (1H, d, J=11.6 Hz, 7-H), 7.06 (1H, d, J=11.6 Hz, 6-H);
MSm/z (%) : 452($M^+$, 32), 434(17), 414(21), 396(32), 378(13), 323(55), 305(18), 285(25), 267(39), 265(20), 135 (100);
UV $\lambda_{max}$ (EtOH): 270.4 nm.

## Claims

1. A process for producing hydroxyvitamin D derivatives (provided that vitamin D derivatives in which only the 25-position is converted into a hydroxyl group are excluded), **characterized by** converting a hydrogen atom or atoms at the 2-position, 24-position, 25-position and/or 26-position of a vitamin D into a hydroxyl group or groups in a solution containing a microorganism which can hydroxylate vitamin Ds or an enzyme produced by that microorganism.

2. The process for producing hydroxyvitamin D derivatives according to claim 1, wherein a cyclodextrin is present together with the vitamin D in the solution containing the microorganism capable of hydroxylating vitamin Ds or the enzyme produced by that microorganism.

3. The process for producing hydroxyvitamin D derivatives according to claims 1 or 2, wherein the microorganism capable of hydroxylating vitamin Ds belongs to the genus *Nocardia, Streptomyces*, *Sphingomonas* or *Amycolata.*

4. The process for producing hydroxyvitamin D derivatives according to any one of claims 1 to 3, **characterized by** converting the hydrogen atom or atoms at the 2-position or 2-position and 25-position of the vitamin D into a hydroxyl group or groups.

5. The process for producing hydroxyvitamin D derivatives according to any one of claims 1 to 3, **characterized by** converting the hydrogen atom at the 24-position of the vitamin D into a hydroxyl group.

6. The process for producing hydroxyvitamin D derivatives according to any one of claims 1 to 3, **characterized by** converting the hydrogen atom at the 26-position of the vitamin D into a hydroxyl group.

7. A vitamin $D_3$ derivative represented by the formula (I):

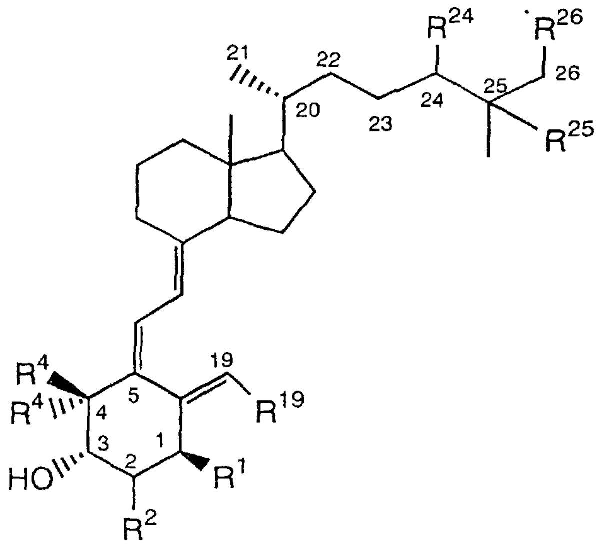

(I)

wherein $R^1$, $R^2$, $R^{24}$, $R^{25}$ and $R^{26}$ each independently represents a hydrogen atom or a hydroxyl group; and $R^4$ and $R^{19}$ each independently represents a hydrogen atom or a fluorine atom, provided that one of $R^2$, $R^{24}$ and $R^{26}$ is a hydroxyl group, and the others are each a hydrogen atom, provided that 1α,24-dihydroxyvitamin $D_3$ wherein $R^1$ and $R^{24}$ each represents a hydroxyl group, and $R^2$, $R^4$, $R^{19}$, $R^{25}$ and $R^{26}$ each represents a hydrogen atom is excluded.

**8.** The vitamin $D_3$ derivative according to claim 7, wherein the vitamin $D_3$ derivative represented by the formula (I) is:

(1) 1α,26-dihydroxyvitamin $D_3$,
(2) 2α,25-dihydroxyvitamin $D_3$,
(3) 19-fluoro-24-hydroxyvitamin $D_3$,
(4) 19-fluoro-26-hydroxyvitamin $D_3$,
(5) 19-fluoro-1α,24-dihydroxyvitamin $D_3$,
(6) 19-fluoro-1α,26-dihydroxyvitamin $D_3$,
(7) 4,4-difluoro-24-hydroxyvitamin $D_3$,
(8) 4,4-difluoro-26-hydroxyvitamin $D_3$,
(9) 4,4-difluoro-1α,24-dihydroxyvitamin $D_3$, or
(10) 4,4-difluoro-1α,26-dihydroxyvitamin $D_3$.

## INTERNATIONAL SEARCH REPORT

International application No.
PCT/JP00/02410

**A. CLASSIFICATION OF SUBJECT MATTER**

Int.Cl$^7$ C12P 7/02, C07C 401/00 // (C12P 7/02, C12R 1:365), (C12P 7/02, C12R 1:465), (C12P 7/02, C12R 1:01)

According to International Patent Classification (IPC) or to both national classification and IPC

**B. FIELDS SEARCHED**

Minimum documentation searched (classification system followed by classification symbols)
Int.Cl$^7$ C12P 7/02, C07C 401/00

Documentation searched other than minimum documentation to the extent that such documents are included in the fields searched

Electronic data base consulted during the international search (name of data base and, where practicable, search terms used)
REGISTRY(STN),CA(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP, 4-356190, A (Taisho Pharmaceutical Co., Ltd.), 09 December, 1992 (09.12.92) (Family: none) | 1,3,5-8<br>2 |
| X | EP, 298757, A (Taisho Pharmaceutical Co., Ltd.), 11 January, 1989 (11.01.89)<br>& JP, 2-469, A & US, 4892821, A<br>& DE, 3884870, G & ES, 2047033, T3 | 1,3 |
| X | JP, 2-231089, A (Taisho Pharmaceutical Co., Ltd.), 13 September, 1990 (13.09.90) (Family: none) | 1,3 |
| X | JP, 7-241197, A (KYOWA HAKKO KOGYO CO., LTD.), 19 September, 1995 (19.09.95) (Family: none) | 1,3 |
| X<br>Y | EP, 461921, A (Mercian Corporation), 18 November, 1991 (18.11.91)<br>& JP, 4-166090, A & CA, 2044550, A<br>& TW, 234147, A & DE, 69112716, E<br>& US, 5474923, A & ES, 2079573, T3 | 1-3<br>2 |
| X | JP, 6-205685, A (Sumitomo Chemical Company, Limited), | 1,5,7 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 May, 2000 (29.05.00) | 06 June, 2000 (06.06.00) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP00/02410

C (Continuation). DOCUMENTS CONSIDERED TO BE RELEVANT

| Category* | Citation of document, with indication, where appropriate, of the relevant passages | Relevant to claim No. |
|---|---|---|
| | 26 July, 1994 (26.07.94) (Family: none) | |
| X | JP, 5-153980, A (Sumitomo Chemical Company, Limited), 22 June, 1993 (22.06.93) (Family: none) | 1,5,7 |
| X | JP, 50-100043, A (Masayuki Ishikawa), 08 August, 1975 (08.08.75) (Family: none) | 7 |
| X | US, 4011250, A (Masayuki Ishikawa), 08 March, 1977 (08.03.77) & JP, 51-019752, A & DE, 2535308, A | 7 |
| X | POSNER, G. H. et al., "Stereocontrolled synthesis of a trihydroxylated A ring as an immediate precursor to 1α,2α,25-trihydroxyvitamin D3", J. Org. Chem. (1991) Vol.56, No.14, p.4339-4341 | 7 |
| X | WO, 98/51678, A1 (WOMEN & INFANT'S HOSPITAL), 19 November, 1998 (19.11.98) & EP, 981523, A1 & AU, 9874936, A | 7 |
| X | WO, 84/01577, A (STARING E.G.J.), 26 April, 1984 (26.04.84) & JP, 60-500133, W & NL, 8204070, A & EP, 122279, A | 7 |
| X | JP, 52-057156, A (Teijin Limited), 11 May, 1977 (11.05.77) (Family: none) | 7 |
| X | JP, 53-026313, A (Teijin Limited), 11 March, 1978 (11.03.78) (Family: none) | 7 |

Form PCT/ISA/210 (continuation of second sheet) (July 1992)